**Europäisches Patentamt**

**European Patent Office**

**Office européen des brevets**

(19)

(11) Publication number: **0 180 559**
**A2**

# EUROPEAN PATENT APPLICATION

(12)

(21) Application number: **85850280.0**

(22) Date of filing: **10.09.85**

(51) Int. Cl.⁴: **A 61 K 7/48**

(30) Priority: **23.10.84 US 663791**

(43) Date of publication of application: **07.05.86**
**Bulletin 86/19**

(84) Designated Contracting States: **DE FR GB**

(71) Applicant: **Orentreich, Norman, 909 Fifth Avenue, New York New York 10021 (US)**

(72) Inventor: **Orentreich, Norman, 909 Fifth Avenue, New York New York 10021 (US)**

(74) Representative: **Bergvall, Stina-Lena, Dr. Ludwig Brann Patentbyrä AB Box 7524 Kungsgatan 3, S-103 92 Stockholm (SE)**

(54) **Skin moisturizing composition and method.**

(57) A skin moisturizing composition comprising an effective amount of trehalose may be applied to dry or drying areas of animal skin to inhibit or treat skin dryness. The trehalose is applied in a dermatologically acceptable carrier which may include other humectants, such as glycerin or other polyol, emollients and/or barrier agents. The trehalose is present in the compositions of the invention in an amount of about 0.1 to 20 weight percent, preferably about 1 to 10 weight percent, and may be applied in conventional formulations such as creams, lotions, ointments, balms, milks, tinctures or gels.

0180559

## SKIN MOISTURIZING COMPOSITION AND METHOD

### Field of the Invention

The present invention relates to a skin moisturizing composition and method for inhibiting or treating skin dryness. More particularly, the invention is directed to the use of trehalose as an aid in skin moisturizing and inhibiting or treating skin dryness.

### Background of the Invention

The normal state of skin hydration is determined by three factors or circumstances:

1. The ability of skin, particularly the outer layer (stratum corneum) of the epidermis, to retain water;

2. The speed with which water is eliminated by evaporation, referred to as transepidermal water loss (TEWL); and

3. The speed with which water moves from lower layers to upper layers of the skin.

Each circumstance is dependent upon the other, while the complete state of skin hydration is dependent upon the natural moisturizing factors (NMF), phospholipids and mucopolysaccharides. The NMF consists of 2-pyrrolidone-5-carboxylic acid, urea, ammonia, glucosamines, creatinine, uric acid, ions (chlorides, sodium, potassium), lactates, formates, citrates and unidentified substances and proteins comprising up to about 40 percent of NMF. It is the water-fixative capacity and humectancy of the skin that relies on the integrity of NMF, phospholipids and mucopolysaccharides. That integrity may be compromised during the aging process or due to nutritional imbalances or exposure to adverse conditions. In any case, the dry skin state results from decreases in the water fixation capacity of the skin.

Skin is a protective organ whose prime function is to produce a stratum corneum protective layer. This does not always occur optimally, and many skin problems are caused by and have associated with them a dry, disrupted, flaky, scaly, irregular, discontinuous surface. This large category of skin disorders (referred to generally as xerodermas) includes such conditions as icthyosis, psoriasis, windburn, atopic dermatitis, eczema, pruritus hiemalis, etc. With aging, the problem of imperfect keratinization can produce a constant dry skin state with marked itching (senile pruritus).

Traditionally, it was thought that emollients, such as fats, phospholipids, sterols and the like were the essential substances necessary for maintenance or restoration of skin softness and flexibility. More recently however, it has been found that dry skin conditions result largely from the loss of water-soluble natural substances

0180559

(NMF) from the skin. Although the stratum corneum is somewhat hygroscopic and absorbs water vapor, the loss of NMF inhibits the ability of the stratum corneum to absorb and retain sufficient moisture for restoration to a normal condition. This creates the need for artificial moisturizing systems, which conventionally have attempted to hydrate the skin through oil/water emulsion techniques. See, for example, U.S. Patents Nos. 3,957,971 and 4,151,304.

### Summary of the Invention

According to the present invention it has been found that dry skin can be treated and/or skin drying inhibited by the topical application of a skin moisturizing composition comprising an effective amount of trehalose. The trehalose may be applied in a dermatologically acceptable carrier or vehicle, and is particularly effective when incorporated in the usual water/oil emulsions (creams, lotions, ointments, balms, milks, etc.) commonly used for skin moisturizing purposes. The vehicle may contain moisturizing aids including humectants, emollients and/or barrier agents.

### Brief Description of the Drawings

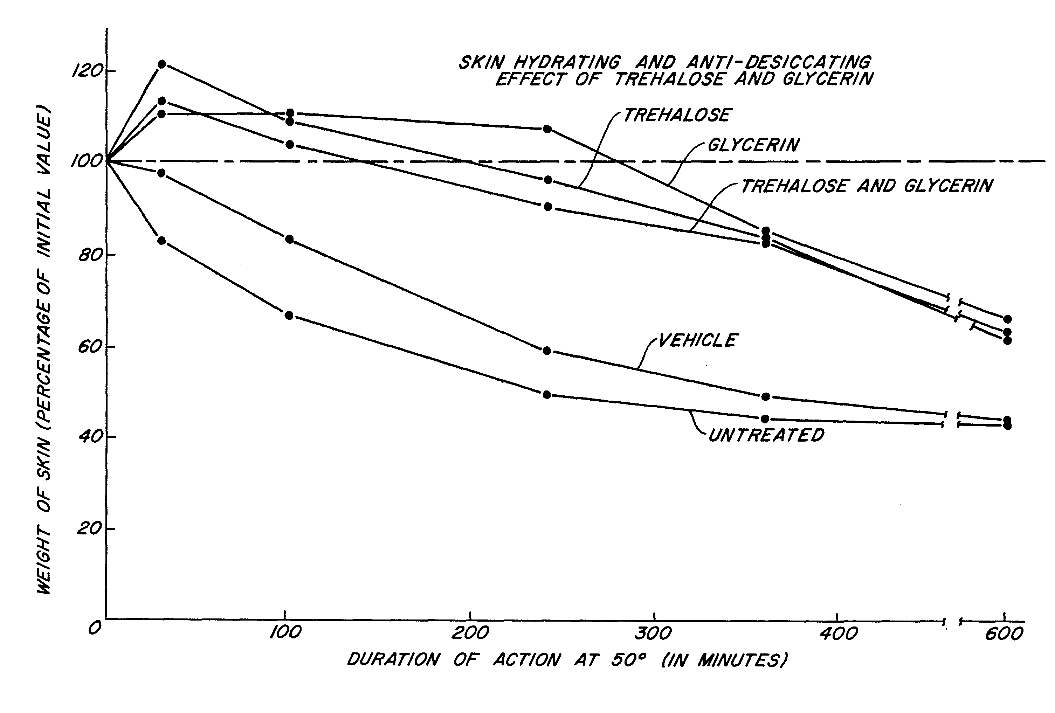

The sole figure of drawings is a graph plotting moisture retention (as a percentage of skin weight) against time, comparing three compositions containing trehalose and/or glycerin with untreated skin and skin treated with vehicle alone, as more fully described in Experimental

Example 1 below.

## Detailed Description of Preferred Embodiments

Trehalose, also known as mycose, is a disaccharide formed of two molecules of glucose. It is found in nature in trehala manna, fungi and certain other organisms. Extraction procedures for obtaining trehalose are well known (see Merck Index Page 1370, No. 9399). Trehalose is commercially available from Pfsanstiehl Laboratory and Company, Waukegan, Illinois, in the form of a white crystalline powder.

Various water-soluble polysaccharides, including glucose and sucrose-type derivatives, have served in the past as emollients, humectants and emulsifiers, and have been used in various cosmetic compositions. For example, methyl glucose ether (also known as methyl glucam and sold under a series of trademarks including "Glucam" and "Amerchol") are glucose type derivatives which have been used as emollients. See also U. S. Patent No. 3,659,025. However, it has not previously been known to use trehalose either alone or in combination with other skin moisturizing aids in skin moisturizing or other skin care treatment products.

The skin moisturizing compositions of the present invention contain about 0.1 to 20 weight percent of trehalose, and preferably about 1 to 10 weight percent trehalose, in a non-toxic dermatologically acceptable carrier or vehicle.  As the vehicle one may use a conventional moisturizing composition or other cosmetic base, preferably one which is non-drying and aides in the moisturizing or hydration of the skin.

Trehalose is soluble in cold water or hot alcohol, and is easily incorporated into many formulations.  Preferably, trehalose is added to the heated water phase prior to emulsification.  The vehicle may take any of a number of conventional forms, including, for example, creams, lotions, ointments, balms, milks, tinctures and gels.  Such vehicles may contain emollients, humectants, barrier agents, emulsifiers, healing agents, preservatives, and other ingredients, as desired, all of which are well known in the art, and representative ingredients are included in the Formulation Example below.  Suitable preservatives include methyl paraben, propyl paraben, imidazolidinyl urea, etc.

Known humectants which may be used in compositions according to the present invention include up to about 10 weight percent of a polyol (polyhydric alcohol), such as glycerin (glycerol), propylene glycol, butylene glycol, sorbitol and/or others. The glycerin or other polyol may be present in an amount of about 2 to 4 weight percent of the composition or about a 1:1 to 2:1 molar ratio with the trehalose (i.e., about 25 to 50 percent by weight of the trehalose).  While applicant does not wish to be bound by any particular theory, it is believed trehalose dihydrate

0180559

forms a complex (glyceride) with glycerin displacing one or both of the water moieties.

For usual applications a preferred embodiment may contain about 4 percent trehalose, while for difficult applications a preferred embodiment may contain about 8 percent trehalose.

Examples of suitable composition formulations according to the present invention are set forth as Examples 1-5 below. It will be understood that these formulations are merely exemplary and are not intended to be exhaustive or limiting in any manner.

0180559

## FORMULATION EXAMPLE 1

### Topical Cream

| Ingredient | %w/w |
|---|---|
| glyceryl monostearate | 2.0 |
| stearic acid | 2.0 |
| mineral oil | 5.0 |
| sesame oil | 5.0 |
| cetyl alcohol | 4.0 |
| lanolin oil | 2.0 |
| isopropyl myristate | 3.0 |
| triethanolamine | 1.0 |
| magnesium aluminum silicate | 1.0 |
| glycerin | 4.0 |
| trehalose | 4.0 |
| preservative | QS |
| water | 62.0 |
| | 100.0 |

## FORMULATION EXAMPLE 2

### Topical Ointment

| Ingredient | %w/w |
|---|---|
| trehalose | 8.0 |
| glycerin | 2.0 |
| cholesterol | 2.0 |
| mineral oil | 10.0 |
| petrolatum | 78.0 |
| | 100.0 |

## FORMULATION EXAMPLE 3

### Lip Balm

| Ingredient | %w/w |
|---|---|
| trehalose | 2.0 |
| castor oil | 40.0 |
| isopropyl palmitate | 10.0 |
| white wax | 10.0 |
| carnuba wax | 5.0 |
| ozokerite wax | 5.0 |
| preservative | QS |
| stearic aid | 5.0 |
| petrolatum | 23.0 |
| | 100.0 |

## FORMULATION EXAMPLE 4

### Skin Milk

| Ingredient | %w/w |
|---|---|
| glyceryl monostearate | 3.0 |
| isopropyl isostearate | 8.0 |
| sesame oil | 4.0 |
| trehalose | 5.0 |
| glycerin | 3.0 |
| carbomer 940* | 0.2 |
| triethanlamine | 0.15 |
| preservative | QS |
| water | 76.65 |
| | 100 |

* Water soluble vinyl polymer of B.F. Goodrich Company (Carbopal trademark) used as suspending, thickening or gel-forming agent.

0180559

FORMULATION EXAMPLE 5

Aerosol Lotion

| Ingredient | %w/w |
|---|---|
| glyceryl monostearate | 2.0 |
| lanolin alcohols | 2.0 |
| myristyl myristate | 1.5 |
| mineral oil | 4.0 |
| polyectylene 50 monostearate | 2.0 |
| water | 10.0 |
| trehalose | 2.5 |
| alcohol | 40.0 |
| methyl chloride | 10.0 |
| isobutane | 6.0 |
| chlorohydrocarbon propellant | 20.0 |
| | 100.0 |

The compositions of the present invention are applied to the dry or drying areas of the skin in the same manner as conventional skin moisturizing formulations, preferably twice a day such as in the morning and in the evening. While applicant does not wish to be bound by any particular theory, it is believed that the compositions of the present invention ameliorate the abnormally dry skin state by slowing the deleterious effects of protein breakdown in the skin. That is, in much the same way as occurs in animal forms which undergo cryptobiosis, protein

11    0180559

molecules are kept intact because the trehalose takes the place of water, thus preventing protein denaturation. In other words, trehalose is believed to act as a water substitute to prevent further protein breakdown.

The beneficial effects of the present invention will now be demonstrated with reference to the following experimental example in which the skin hydrating and anti-desiccation effects of a composition according to the invention were measured quantatively.

EXPERIMENTAL EXAMPLE 1

The moisturizing and anti-desiccating effects of a trehalose and/or glycerin were evaluated in the laboratory using the technique of H. Lauressergues (1983) at the Centre de Recherches Pierre Fabre, France. Intact stratum corneum (SC) was obtained by placing rat tails in a watertight plastic container and immersing it for 90 sec. in a 70 degrees C waterbath. The entire SC was removed by pulling the stratum corneum manually.

The test preparations included (1) 4 weight percent trehalose in vehicle, (2) 4 weight percent glycerin in vehicle, (3) a complex of 4 weight percent trehalose and 4 weight percent glycerin in vehicle, and (4) vehicle alone. The vehicle consisted of an oil phase containing:

Amerchol L101

Sesame Oil

Cetyl alcohol Isostearyl neo pentanoate

Stearic acid

Glyceryl monostearate

Ritaderm

Propyl paraben and a water phase containing:

Water

Trehalose dihydrate

Glycerin

Triethanolomine

Magnesium aluminum silicate

Lecithin

Methyl paraben

Samples of SC measuring 1-1.5 $cm^2$ were weighed immediately and placed over a glass slide with the dermal side down. The test preparations, also of known weight, were each spread over the SC surface. On a separate slide, an equivalent weight of test preparation was spread over the same area of the glass slide. SC without any moisturing preparation was also placed on glass slides for comparison.

All samples were placed in an incubator at 50 degrees C and a humidity of 25%. The weight of the samples was determined at specified time intervals. The weight of the SC at any given time was determined by subtracting the equivalent weight of the test preparation alone, assuming that the rate of water loss is similar in both systems.

the amount of evaporative water loss was calculated by subtracting the SC weight at any given time from the inital SC weight.

In the preparation of the SC, the brief immersion at 70 degrees C caused some loss of water. When the test moisturizing preparations are applied some of the water from the preparation is incorporated into the SC. This accounts for the initial increase in weight of the SC and agrees well with the criteria for moisturizing effect described by Lauressegues. The trehalose in the preparation prevents the otherwise rapid evaporative water loss normally expected in unprotected SC.

The accompanying figure shows the following:

1.    The untreated or unprotected skin loses water at a relatively constant rate.

2.    The skin treated with the vehicle alone without trehalose and/or glycerin possesses little moisturizing or antidehydration effect.

3.    The skin treated with trehalose and/or glycerin initially increases in weight (i.e., increase in water content) which shows their moisturizing properties.

4.    The dehydrating effect of the dry heat condition was greatly retarded when rat tail skin was treated with a preparation containing trehalose and/or glycerin.

14    0180559

It will be recognized by those skilled in the art that changes may be made to the above-described embodiments of the invention without departing from the broad inventive concepts thereof. It is understood, therefore, that this invention is not limited to the particular embodiments disclosed, but it is intended to cover all modifications which are within the scope and spirit of the invention as defined by the appended claims.

C180559

## CLAIMS

1. A skin moisturizing composition comprising an effective amount of trehalose and a dermatologically acceptable carrier.

2. A composition according to Claim 1 wherein trehalose is present in an amount of about 0.1 to 20 weight percent of the composition.

3. A composition according to Claim 1 wherein trehalose is present in an amount of about 1 to 10 weight percent of the composition.

4. A. composition according to Claim 1 wherein said composition contains a polyol.

5. A composition according to Claim 1 wherein said polyol is glycerin.

6. A composition according to Claim 4 wherein said polyol is present in an amount of about 1 to 10 weight percent of the composition.

7. A composition according to Claim 4 wherein said polyol and trehalose are present in a molar ratio of about 1:1.

8. A composition according to Claim 4 wherein said polyol is present in an amount of about 2 to 4 weight percent.

9. A composition according to Claim 1 wherein said dermatologically acceptable carrier comprises at least one ingredient selected from the group consisting of

0180559

emollients, humectants and barrier agents.

10. A composition according to Claims 1 wherein said dermatologically acceptable carrier is selected from the group consisting of creams, lotions, ointments, balms and milks.

11. A method of inhibiting or treating animal skin dryness comprising applying topically to the area of dry or drying skin an effective amount of trehalose.

12. A method according to Claim 11 wherein trehalose is applied to the skin in a composition containing a polyol.

13. A method according to Claim 11 wherein trehalose is applied to the skin in a dermatologically acceptable carrier.

14. A method according to Claim 13 wherein said carrier includes at least one ingredient selected from the group consisting of emollients, humectants and barrier agents.

SKIN HYDRATING AND ANTI-DESICCATING
EFFECT OF TREHALOSE AND GLYCERIN

TREHALOSE

GLYCERIN

TREHALOSE AND GLYCERIN

VEHICLE

UNTREATED

WEIGHT OF SKIN (PERCENTAGE OF INITIAL VALUE)

DURATION OF ACTION AT 50° (IN MINUTES)

1/1

0180559